# EUROPÄISCHE PATENTSCHRIFT

(11) **EP 0 815 898 B1**
(45) Veröffentlichungstag und Bekanntmachung des Hinweises auf die Patenterteilung: **08.01.2003**
(21) Anmeldenummer: 97111077.0
(22) Anmeldetag: 02.07.1997
(51) Int. Cl.: A61N 1/16

(54) **Elektromagnetische Abschirmung für Gebäude**
Electromagnetic shielding for buildings
Blindage électromagnétique pour bâtiments

(30) Priorität: 04.07.1996 DE 29611617 U; 26.06.1997 DE 29711054 U
(43) Veröffentlichungstag der Anmeldung: 07.01.1998
(73) Patentinhaber: MARBURGER TAPETENFABRIK J.B. SCHAEFER GMBH & CO. KG, D-35274 Kirchhain (DE)
(72) Erfinder: Eitel, Ullrich, 35039 Marburg (DE); Stolzenberg, Peter G, 26125 Oldenburg (DE)
(74) Vertreter: Olbricht, Karl Heinrich, Dipl.-Phys.

(56) Entgegenhaltungen:
- CH-A- 671 885
- DE-A- 3 611 675
- DE-A- 3 707 238
- DE-A- 4 026 403
- DE-U- 8 224 462

## Beschreibung

Die Erfindung betrifft eine Abschirmung gemäß dem Oberbegriff von Anspruch 1.

Es ist häufig erwünscht und manchmal notwendig, bestimmte Gebäude, Räume, Schränke o.dgl. vor elektromagnetischen Wellen und Feldern zu schützen. Derartige Abschirmungen sind beispielsweise im klinischen Bereich in Räumen erforderlich, wo empfindliche Untersuchungen vorgenommen werden, namentlich mit EKG- und EEG-Geräten (Elektrokardiographen bzw. Elektroenzephalographen). Man benötigt Abschirmungen ferner in Räumen, die abhörsicher sein müssen. Auch gibt es in der Bevölkerung einen Anteil besonders sensibler Personen, deren Wohlbefinden durch elektromagnetische Einflüsse im Wohn- und Aufenthaltsbereich mehr oder weniger stark beeinträchtigt werden kann, z.B. in der Nähe von Umspannwerken, Radarstationen, Funk- oder Fernseh-Sendern usw.

Typische Raumschirmungen bedienen sich einfacher Metallgitter oder -netze, die nach dem Prinzip des Faraday-Käfigs ein feldfreies Rauminneres bewirken, ohne besondere Maßnahmen aber wenig wasserdampf- und oxidationsbeständig sind. In der DE-A-3 707 238 wird eine Matte mit einer Aluminiumfolie auf einer Kunststoff-Schaumschicht vorgeschlagen, die ein leitfähiges Pulver enthält. Eine gemäß DE-U-8 224 462 ausgebildete Abschirmfolie hat Ausnehmungen, die gleichmäßig oder ungleichmäßig verteilt sein können. Vorgefertigte Folienstreifen aus elektrisch und/oder magnetisch leitendem Material können, wie aus DE-U-9 105 843 hervorgeht, über vorhandene elektrische Leitungen geklebt werden, die unter oder auf Putz verlegt sind. Den bekannten Vorrichtungen ist gemeinsam, daß sie materialbedingt verhältnismäßig großes Gewicht haben, wodurch die Anbringung gewisse Baumaßnahmen erfordert; nicht überall kann man sie einsetzen. Vielfach stört die mangelhafte Atmungseigenschaft ausgekleideter Räume.

Wie eine normale Tapete ist eine sog. SHIELDEX-Raumschirmung verlegbar, die durch ein verkupfertes und/oder versilbertes Nylonfaser-Vlies gekennzeichnet ist. Für Fenster sind Jalousien aus diesem Material vorgesehen, mit dem auch Türen überzogen werden. Türschwelle und Zarge sind mit besonderen EMV-Dichtungen versehen, wodurch weitgehende Dichtigkeit für elektromagnetische Wellen gesichert werden soll. Elektrische Leitungen in oder aus dem geschirmten Raum hat man gemäß DE-B-4 311 125 mit Filtern ausgestattet. Einrichtungen dieser Art erfordem einen hohen technischen Aufwand und entsprechend hohen Preis.

Eine Abschirmung für Gebäude, Räume, Schränke u.dgl. gemäß US-A-4 965 408 benutzt einen an raumbegrenzenden Wänden flächig anbringbaren Träger, der ein klebbares Vlies und eine leitfähige Schicht in Form einer Aluminiumfolie aufweist, auf der eine isolierende Deckschicht aufgebracht ist. Bei vollflächiger Abdeckung lassen sich Folienknicke insbesondere an Ecken, Winkeln und Krümmungen nicht vermeiden, so daß Störungen der Abschirmung elektrischer und/oder magnetischer Felder nicht auszuschließen sind. Beim Entfernen der Polymer-Deckschicht von der Abschirmfolie wird diese jeweils vollständig freigelegt; sie kann danach wieder mit neuem Deckmaterial überklebt werden. Wiederholt man das öfter, so sind Beschädigungen der Abschirmung kaum vermeidbar. Reparaturen an der dünnen Alufolie sind jedoch aufwendig und unsicher.

Hiervon ausgehend stellt sich die Aufgabe einer nachhaltigen Verbesserung. Es ist Ziel der Erfindung, mit möglichst geringem wirtschaftlichem Aufwand eine Abschirmung zu schaffen, die zuverlässigen Schutz vor elektromagnetischen Wellen bzw. Feldem gewährleistet und die preiswert beschafft sowie angebracht werden kann.

Diese Aufgabe wird durch die Merkmale des Anspruchs 1 gelöst. Bei einer Abschirmung für Gebäude, Räume, Schränke u.dgl., mit einem an raumbegrenzenden Wänden flächig anbringbaren Träger, der ein klebbares Vlies sowie eine durchgehende leitfähige Schicht aufweist, die eine isolierende Strip-Beschichtung trägt, auf der weitere Schichten aufbringbar und wieder abnehmbar sind, sieht die Erfindung gemäß dem kennzeichnenden Teil von Anspruch 1 vor, daß der Träger als rollbares Flachmaterial in Form klebbarer Tapetenbahnen ausgebildet ist und daß entlang zumindest einer Bahnkante ein vorzugsweise längsperforierter Randbereich unbeschichtet, d.h. frei von einer Strip-Beschichtung ist, so daß die leitfähige Schicht offenliegt. Diese wird auch durch wiederholtes Abziehen einer auf die Strip-Beschichtung aufgebrachten Beschichtung, beispielsweise einer Übertapete, nicht zerstört. Kostenintensive Reparaturen der leitenden Schicht entfallen demnach vollständig. Bei derartigen Trägem kann man auf die Verwendung von massivem Metall verzichten. wodurch man außerordentliche Gewichts-Einsparungen gegenüber herkömmlichen Vorrichtungen erzielt, die zudem meist den Einsatz teurer Metalle wie Kupfer und Silber erfordern. Erfindungsgemäß wird jedoch ein spezifisch leichtes leitendes Material verwendet, das auf dem Trägervlies als durchgehende Schicht aufgebracht und seinerseits durch eine Strip-Beschichtung geschützt ist. Weil entlang zumindest einer Bahnkante ein vorzugsweise längsperforierter Randbereich unbeschichtet ist, so daß die leitfähige Schicht offenliegt, kann man die Trägerbahnen auf Stoß aneinander anschließend verlegen. Die Strip-Beschichtung hat außer der Isolierwirkung den Vorteil, daß weitere Beschichtungen aufgebracht und wieder abgenommen werden können, ohne daß die Schirmungswirkung dadurch prinzipiell beeinflußt wird. Infolgedessen bleibt die elektromagnetische Verträglichkeit (EMV) dauerhaft erhalten. Die aufgebrachte Schirmung zeichnet sich überdies durch ihre zugleich verwirklichte Mehrfachfunktion als Putzbelag, Makulatur, Wechselgrund usw. aus; sie definiert ein festes elektrisches Potential, so daß keine Störpotentiale auftreten können.

Laut Anspruch 2 ist das Vlies des Trägers mit einer flächigen, als Isolierung wirkenden Grundbeschichtung versehen, auf welche die leitfähige Schicht aufgebracht ist, wodurch man hohe Festigkeit bei geringem Flächengewicht erzielt. Die leitfähige Schicht besteht gemäß Anspruch 3 im wesentlichen aus Legierungspulver, Graphit bzw. Ruß o.dgl. Geeignet sind vor allem auch leitfähige Leichtmassen, die z.B. unter den Handelsbezeichnungen Vakuperm, Mumetall, V4A, Karbonyleisen usw. erhältliche Pulver enthalten und silikatisiert sein können, um gegen Korrosion gesichert zu sein. Die Schicht kann nach Anspruch 4 aus einer wäßrigen, Haftmittel enthaltenden Dispersion auf die Grundbeschichtung aufgebracht sein, beispielsweise im Siebdruckverfahren. Die Beschichtung ist dauerhaft stabil, so daß die flexible Abschirmung hohen Beanspruchungen standhält Entgegen früheren Annahmen reicht die Leitfähigkeit von Legierungspulver, Graphit bzw. Ruß o.dgl. absolut aus, um gute Abschirmung sicherzustellen. Die Beschichtungsmasse kann durch Streichen, Drucken oder anderweitig aufgebracht werden. Sie kann unmagnetisch sein und ist korrosionsbeständig sowie biologisch unschädlich. Außer der positiven Ökobilanz ergeben sich die weiteren Vorteile, daß die leitende Beschichtung chemisch neutral und allgemein resistent, ferner schwerentflammbar nach DIN 4102 B1 und auch atmungsaktiv nach DIN 53-122 ist, so daß sie Feuchtigkeitsaustausch selbst unter ungünstigen Klimabedingungen weitestgehend zuläßt.

Die benachbarten Randbereiche zweier Bahnen lassen sich laut Anspruch 5 mit einem Abdeckstreifen von gleichem Schichtaufbau derart überdecken, daß die leitfähigen Schichten miteinander verbunden sind, namentlich in stoffschtüssigem Kontakt und im Einklang mit Anspruch 6 bündig übergreifend. Jeder Spalt zwischen den Abschirmungs-Bahnen wird daher auf kostensparende Weise überbrückt und ein Durchtreten elektromagnetischer Wellen bzw. Felder sehr einfach vermieden; weil keine Lücken auftreten, wird über die ein großflächiger, voller Potentialausgleich erzielt.

Überaus wirtschaftlich ist die Maßnahme von Anspruch 7, wonach die Breite eines entlang der Bahn-Perforation abtrennbaren Randstreifens so bemessen ist, daß er als Abdeckstreifen zwei auf Stoß benachbarte offene Bahnränder lückenlos überdeckt. Auf diese Weise wird eine besondere Erzeugung sowie Bereitstellung von Abdeckstreifen überflüssig und zugleich Materialbahn-Abfall auf ein Minimum reduziert.

Eine bedeutsame Weiterbildung der Erfindung besteht gemäß Anspruch 8 darin, daß z.B. an Randstreifen offenliegende Zonen der leitfähigen Schicht mit flächigen, flexiblen Kontaktelementen leitend verbindbar bzw. verbunden sind, vor allem im Bereich von Öffnungen des abzuschirmenden Raumes und/oder an geerdeten Anschlußdosen z.B. der Stromversorgung oder des Telefonnetzes. Solche Kontaktelemente können gemäß Anspruch 9 flächige Kontaktfedern oder auch leitfähige Dichtungsstreifen sein. Mit ihrer Hilfe wird das Eindringen von Wellen bzw. Feldern auch im Bereich von Öffnungen wirksam unterdrückt, so daß die Abschirmung rundum gesichert ist

Sehr vorteilhaft ist femer die Gestaltung nach Anspruch 10, wobei die Strip-Beschichtung eine glatte Außenfläche aufweist, die ohne weiteres mit handelsüblichen Tapeten und/oder Wandbekleidungen beklebbar ist Außer dem mechanischen Schutz der leitenden Beschichtung gewinnt man durch den Strip-Auftrag die Möglichkeit, anschließend aufgebrachte Tapezierungen mühelos von dem darunter befindlichen Schirmungsträger abziehen zu können.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnung. Darin zeigen:
- Fig. 1: eine Teilansicht einer Rolle von Trägermaterial,
- Fig. 2: eine stark vergrößerte Schnittansicht des Randbereichs eines Trägermaterials,
- Fig. 3: einen perspektivischen Einblick in einen abzuschirmenden Raum,
- Fig. 3a: eine schematisierte Seiten-Schnittansicht entsprechend dem Bereich IIIa in Fig. 3,
- Fig. 4: eine vergrößerte Schnittansicht eines Nahtbereichs und
- Fig. 5: eine schematisierte Seiten-Schnittansicht eines Wandbereichs.

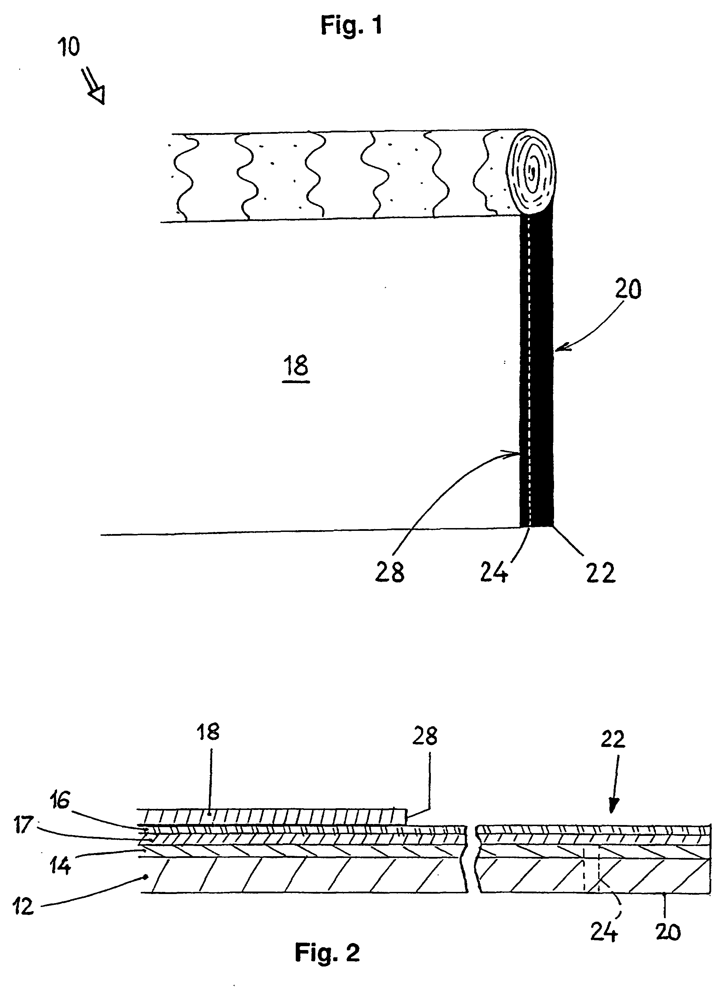

Die schematisierte Darstellung in Fig. 1 läßt einen Träger 10 erkennen, dessen (blanke) Vorderseite von einer Rolle teilweise abgezogen ist. Auf der Rückseite

Gemäß Anspruch 11 ist ferner vorgesehen, daß der Träger z.B. an beschädigten Randbereichen von Hand nachbearbeitbar ist, so daß mit einem Reparatur-Set an lädierten Stellen leitfähige Masse in dispergierter Form aufgepinselt und durch ein Schutzband abgedeckt werden kann.

Zur Steigerung der Dämpfung sieht Anspruch 12 vor, daß zwischen den Deckschichten weitere Schichten mit oder aus Metalllegierungspulver angeordnet sind. Man erzielt dadurch außerdem hohe Festigkeit bei einem Flächengewicht, das im Bereich der üblichen Materialien (z.B. Tapeten) liegt. Die weiteren Schichten wirken als zusätzliche oder Absorptionsschichten bzw. Schichtabsorber. Sie können im Einklang mit Anspruch 13 chemisch neutralisiertes, korrosionsfrei gemachtes Pulver enthalten, das vor allem bei Verwendung von Feinstform-Absorberpigmenten nach Anspruch 14 die Schirmung auch in solchen Frequenzbereichen wesentlich verbessert, in denen die magnetische Wellenkomponente dominiert.

Der Träger kann laut Anspruch 15 in der Art von Radardomen, Moskitonetzen oder ähnlichem selbsttragend und/oder mit Abstützungen einen z.B. zellenförmigen Abschirmbereich bilden, so daß ein Binnenvolumen im Inneren eines Raumes oder auch eine Schirmzone im Freien begrenzbar ist. Dazu läßt sich der einen Membranstoff. eine Vliesbahn o.dgl. aufweisende Träger gemäß Anspruch 16 auch als Vorhang mit decken- und bodenseitigen Abschlüssen ausbilden. kann eine Musterung vorhanden sein, doch soll die Darstellung in Fig. 1 oben nur verdeutlichen, daß es sich um eine aufklebbare Fläche handelt. Während die Vorderseite mit einer glatten Wechselgrund- oder Strip-Beschichtung 18 versehen ist, befindet sich entlang einer Längskante 20 ein Randstreifen 22, der außerhalb einer Strip-Kante 28 frei von einer Strip-Beschichtung ist und eine Perforation 24 aufweist.

Dieser Aufbau geht aus Fig. 2 deutlicher hervor. Man erkennt, daß der Träger 10 ein Vlies 12 mit einer Grundbeschichtung 14 hat, auf der sich eine leitfähige Schicht 16 befindet. Außerhalb des Randstreifens 22 ist die leitfähige, vorzugsweise aus Graphit bestehende oder metallhaltige Schicht 16 durch die Strip-Beschichtung 18 abgedeckt.

In der schematischen Darstellung ist die Dickendimension noch stärker überhöht als das Breitenmaß, um mögliche Proportionen zu veranschaulichen. Die Dicken-Verhältnisse der einzelnen Schichten müssen jedoch der Darstellung nicht entsprechen, wenngleich sie bei einer bevorzugten Ausführungsform des Trägers 10 vorgesehen sind. Beispielsweise sind folgende Schichtdicken zweckmäßig: 160 µm für das Vlies 12; 40 µm für die Grundbeschichtung 14; 20 µm für die leitfähige Schicht 16; und 40 µm für die Strip-Beschichtung 18. Das spezifische Flächengewicht eines solchen Aufbaues liegt bei bloß 140 g/m².

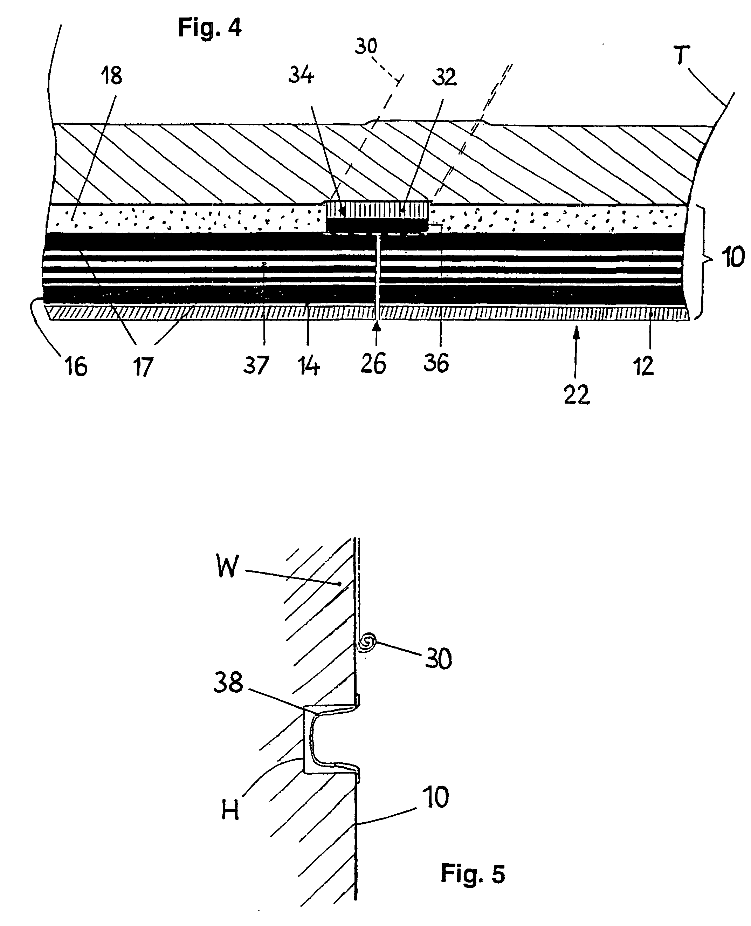

Andere Bemessungen gehen aus Fig. 4 hervor, wo zugleich dargestellt ist, wie zwei auf Stoß nebeneinander liegende Randstreifen 22 im Bereich eines Spalts 26 von einem Abdeckstreifen 30 überbrückt sind. Dieser kann entlang der Perforation 24 von einer Trägerbahn 10 abgetrennt sein, so daß kein Abfall entsteht. Vielmehr benutzt man ein und denselben abgetrennten Streifen zur Stoß- bzw. Naht-Überdeckung mit grundsätzlich gleichartigem Schichtenaufbau, wobei ein Vlies 32 mit einer Grundbeschichtung 34 versehen ist, die eine leitfähige Schicht 36 trägt. In Wirklichkeit ist ein (gestrichelt angedeuteter) Zwischenraum zwischen den leitenden Schichten 16 und 36 nicht vorhanden, weil diese aneinander satt anliegend leitend verbunden sind. Die mit dem Abdeckstreifen 30 lückenlos überdeckten Stoßstellen können weitgehend oder ganz unsichtbar werden, wenn der Träger 10 insgesamt mit einer Übertapete T verkleidet wird. Wichtig ist die Doppelfunktion des Abdeckstreifens 30. Er verhindert einerseits den sog. Schlitzantennen-Effekt und gewährleistet andererseits vollen Potentialausgleich durch die lückenlose leitende Rundum-Schirmung des Raumes.

In Fig. 2 ist eine zusätzliche Deckschicht 17 eingezeichnet, die erhöhte Dämpfung bewirkt. Sie kann bei vereinfachter Ausführung entfallen, doch ist es auch möglich und erfindungsgemäß vorgesehen, eine Anzahl solcher Absorberschichten 17 bzw. 37 (Fig. 4) übereinander anzubringen, beispielsweise karoartig bzw. in abwechselnden Folgen von senkrecht und quer angebrachten Tapezierungen. Man erreicht damit außerordentlich hohe Dämpfungswerte.

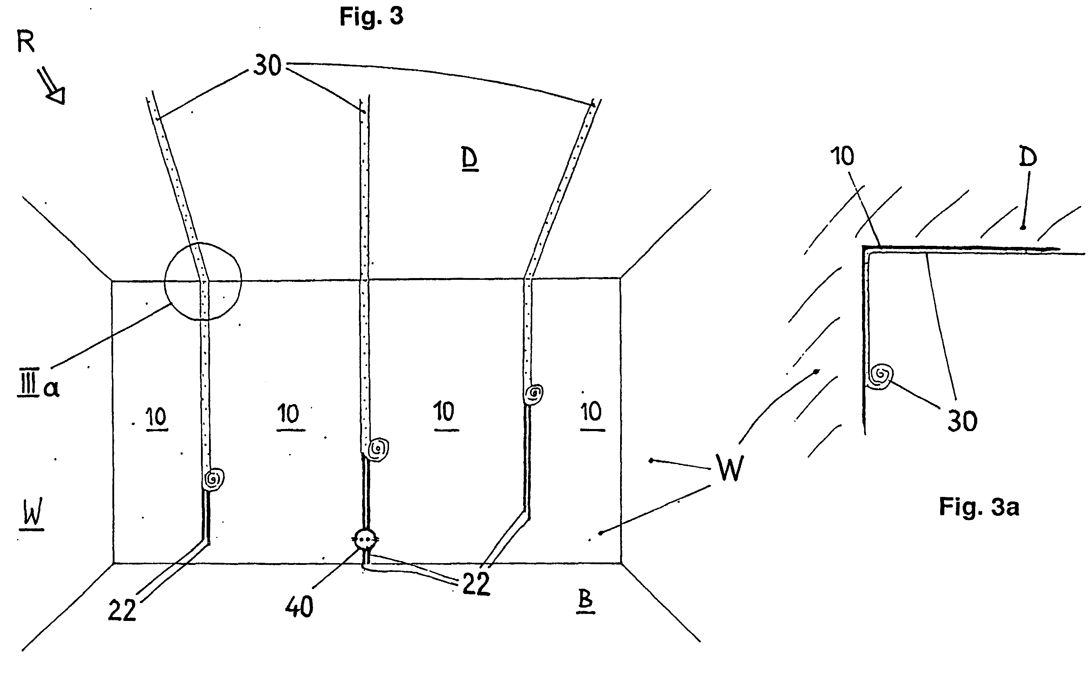

Die Verlegung ist aus den Fig. 3, 3a und 5 ersichtlich. Ein Raum R wird an seinen Wänden W, an der Decke D und am Boden B mit nebeneinanderliegenden Bahnen des Trägers 10 beklebt. Man geht dabei so vor, daß ausgehend von einer Einbaudose 38 (Fig. 5) in einer Höhlung H einer Wand W die Bahnen des Trägers 10 links und rechts auf Stoß anschließend verlegt werden. Die Randstreifen 22 der benachbarten Trägerbahnen 10 werden im Bereich einer Anschlußdose 40 (Fig. 3) durch leitende Verbindung auf Masse- bzw. auf Erdpotential gebracht. Anschließend überzieht ein Abdeckstreifen 30 die offenliegenden Deckschichten 16 bzw. 17 der Randstreifen 22 (Fig. 4). Diese Verbindung kann mit einem Dispersionskleber und durch Anpressen mit einem Nahtroller gesichert werden. Eine Überdeckung von z.B. 3 cm Breite verhindert jedes Klaffen insbesondere in den Kehl-, Eck- und Anschlußbereichen. Zur flächigen Erdung an der gesamten installierten Wand ist zweckmäßig an jeder Wand W mindestens ein Erdungskontakt vorgesehen. Ersatzweise oder zusätzlich läßt sich mit dem Rußabdeckstreifen 30 eine solche Masseverbindung heranführen (Fig. 3 und 3a), was man insbesondere im Bereich der Decke D und auch des Fußbodens machen kann. Praktische Untersuchungen haben gezeigt, daß die erzielte Schirmung von Hochfrequenzfeldern bis um den Faktor 100 besser ist als die Dämpfung einer Stahlbetondecke.

Die Schirmung von Fenstern oder vergleichbaren Öffnungen erfolgt vorzugsweise durch Glas mit ein- oder aufgebrachten Sieben, Metallgittem oder Drahtgeflecht hoher Mesh-Zahl. Weiterhin können solche Öffnungen durch ein mit dem Träger 10 versehenes Rollo geschirmt werden, das im geschlossenen Zustand eine flächige Kontaktierung zur leitfähigen Schicht 16 des Trägers 10 auf der umgebenden Wand W aufweist. Analog schirmt man Türen durch Anbringen des Trägers 10 auf der Tür-Innenseite ab. Im Bereich der Türbänder bzw. -scharniere wird ebenfalls eine flächige, flexible Kontaktierung vorgesehen. Rund um die Türkanten erfolgt die leitende Verbindung mit einem metallischen oder leitend verkleideten Türrahmen über (nicht dargestellte) Kontaktfedern, leitfähige Dichtungen u.dgl., wobei an diesen, Kanten die leitfähige Schicht 16 des Trägers 10 offenliegt.

Die Erfindung ist nicht auf die beschriebenen Ausführungsformen beschränkt. Auch können unterschiedliche Materialien verwendet werden. Während das Vlies 12 typisch aus Zellstoff- und Polyesterfasern besteht, wird die Strip-Beschichtung bevorzugt aus bzw. mit Kunststoff hergestellt; dafür kann ein Polymer eingesetzt werden. Wegen günstiger Oberflächen-Eigenschaften eignet sich Polyvinylchlorid (PVC) mit passend eingestelltem Anteil von Weichmachern, z.B. Phthalaten. Alternativ können auch Beschichtungen aus weichmacher- und PVC-freien Stoffen verwendet werden, z.B. aus einer Strukturmasse gemäß EP-A-0 564 712. Mineralische Füllstoffe und sonstige Zusätze können wichtige Eigenschaften wie Scheuerbeständigkeit, Kratzfestigkeit, Formhaltigkeit usw. maßgeblich beeinflussen. Während die oberste und unterste Schicht durch die Strip-Beschichtung 18 bzw. durch das Vlies 12 gegen Korrosion und mechanische Beschädigung geschützt werden, trägt die Sandwich-Anordnung zusätzlicher oder Hilfs-Absorberschichten 17; 37 von z.B. bis zu 25 µm Dicke erheblich zur Steigerung der Dämpfung auch in magnetisch dominierten Frequenzbereichen bei. Die Partikelgrößen von Legierungs- oder Metallpulvern liegen allgemein im Mikround Submikrobereich, d.h. in der Größenordnung zwischen 1 µm und 1 nm.

Zusammenfassend ist festzuhalten, daß eine Abschirmung für Gebäude, Räume, Schränke u.dgl. erfindungsgemäß von einem die raumbegrenzenden Wände W überziehenden metallhaltigen oder metallfreien Träger 10 gebildet wird, der ein klebbares Vlies 12 mit einer Grundbeschichtung 14 und wenigstens eine leitfähige Schicht 16 sowie eine isolierende Strip-Beschichtung 18 hat. Die leitfähige Schicht 16 kann eine Leichtmasse mit bzw. aus Legierungspulver, Graphit bzw. Ruß o.dgl. enthalten und ist z.B. durch Siebdruck aus einer wäßrigen, Haftmittel enthaltenden Dispersion auf der Grundbeschichtung 14 abgeschieden. Der bevorzugt als klebbare Tapete ausgebildete Träger 10 ist entlang eines längsperforierten Randstreifens 22 unbeschichtet, d.h. frei von einer Strip-Beschichtung 18, so daß die leitfähige Schicht 16 offenliegt. Auf Stoß nebeneinanderliegende Bereiche von Bahnen 10, insbesondere Randstreifen 22, sind mit einem Abdeckstreifen 30 von gleichem Schichtaufbau bündig überdeckbar, wodurch die Beschichtungen 16,17; 36, 37 stoffschlüssig und leitend miteinander verbunden sind. An Randbereichen offenliegende Zonen der bzw. jeder leitfähigen Schicht sind mit flächigen, flexiblen Kontaktelementen leitend verbindbar, vor allem im Bereich von Öffnungen des abzuschirmenden Raumes und/oder an geerdeten Anschlußdosen 40 z.B. der Stromversorgung oder des Telefonnetzes. Die Strip-Beschichtung 18 hat eine glatte, mit Tapeten T und/oder Wandbekleidungen beklebbare Außenfläche.

### Bezugszeichenliste

- B: Boden
- D: Decke
- H: Höhlung
- R: Raum
- T: Übertapete
- W: Wände

- 10: Träger
- 12: Vlies
- 14: Grundbeschichtung
- 16: leitfähige Schicht/Flächengebilde
- 17: Deckschicht(en)
- 18: Strip-Beschichtung
- 20: Bahnkante
- 22: Randstreifen / -bereich
- 24: Perforation
- 26: Stoß/Naht
- 28: Strip-Kante
- 30: Abdeckstreifen
- 32: Vlies
- 34: Grundbeschichtung
- 36: leitfähige Schicht
- 37: Absorberschicht (en)
- 38: Einbaudose
- 40: Anschlußdose

## Patentansprüche

1. Abschirmung für Gebäude, Räume, Schränke u.dgl., mit einem an raumbegrenzenden Wänden (W, B, D) flächig anbringbaren Träger (10), der ein klebbares Vlies (12) sowie eine durchgehende leitfähige Schicht (16) aufweist, die eine isolierende Strip-Beschichtung (18) trägt, auf der weitere Schichten aufbringbar und wieder abnehmbar sind, **dadurch gekennzeichnet, daß** der Träger (10) als rollbares Flachmaterial in Form klebbarer Tapetenbahnen ausgebildet ist und daß entlang zumindest einer Bahnkante (20) ein vorzugsweise längsperforierter Randbereich (22) unbeschichtet, d.h. frei von einer Strip-Beschichtung (18) ist, so daß die leitfähige Schicht (16) offenliegt.

2. Abschirmung nach Anspruch 1, **dadurch gekennzeichnet, daß** das Vlies (12) des Trägers (10) mit einer flächigen, als Isolierung wirkenden Grundbeschichtung (14) versehen ist, auf welche die leitfähige Schicht (16) aufgebracht ist.

3. Abschirmung nach Anspruch 1 oder 2, **dadurch gekennzeichnet, daß** die leitfähige Schicht (16) aus Legierungspulver, Graphit bzw. Ruß besteht.

4. Abschirmung nach Anspruch 2 und 3, **dadurch gekennzeichnet, daß** die leitfähige Schicht (16) aus einer wäßrigen, Haftmittel enthaltenden Dispersion auf die Grundbeschichtung (14) aufgebracht ist, z.B. durch Siebdruck.

5. Abschirmung nach einem der Ansprüche 1 bis 4, **dadurch gekennzeichnet, daß** benachbarte Randbereiche (22) zweier Bahnen (10) mit einem Abdeckstreifen (30) von gleichem Schichtaufbau derart überdeckbar sind, daß die leitfähigen Schichten (16, 36) miteinander leitend verbunden sind, namentlich in stoffschlüssigem Kontakt.

6. Abschirmung nach Anspruch 5, **dadurch gekennzeichnet, daß** auf Stoß nebeneinanderliegende Bahnränder von dem Abdeckstreifen (30) bündig übergreifbar bzw. überdeckt sind.

7. Abschirmung nach Anspruch 6, **dadurch gekennzeichnet, daß** die Breite eines entlang der Perforation (24) abtrennbaren Randstreifens (22) so bemessen ist, daß er als Abdeckstreifen (30) zwei auf Stoß benachbarte offene Bahnränder lückenlos überdeckt.

8. Abschirmung nach einem der Ansprüche 1 bis 7, **dadurch gekennzeichnet, daß** z.B. an Randstreifen (22) offenliegende Zonen der leitfähigen Schicht (16) mit flächigen, flexiblen Kontaktelementen leitend verbindbar bzw. verbunden sind, vor allem im Bereich von Öffnungen des abzuschirmenden Raumes und/oder an geerdeten Anschlußdosen (40) z.B. der Stromversorgung oder des Telefonnetzes.

9. Abschirmung nach Anspruch 8, **dadurch gekennzeichnet, daß** die Kontaktelemente als flächige Kontaktfedern und/oder als leitfähige Dichtungsstreifen ausgebildet sind.

10. Abschirmung nach einem der Ansprüche 1 bis 9, **dadurch gekennzeichnet, daß** die Strip-Beschichtung (18) eine glatte Außenfläche aufweist, die namentlich mit einer Übertapete (T) oder Plakaten beklebbar ist.

11. Abschirmung nach einem der Ansprüche 1 bis 10, **dadurch gekennzeichnet, daß** der Träger (10) z.B. an beschädigten Randbereichen von Hand nachbearbeitbar ist.

12. Abschirmung nach einem der Ansprüche 5 bis 11, **dadurch gekennzeichnet, daß** zwischen Deckschichten (16, 36) weitere Schichten mit oder aus Metallegierungspulver (17) angeordnet sind.

13. Abschirmung nach Anspruch 12, **dadurch gekennzeichnet, daß** die weiteren Schichten chemisch neutralisiertes, korrosionsfreies Metallegierungspulver enthalten.

14. Abschirmung nach Anspruch 12 oder 13, **dadurch gekennzeichnet, daß** die weiteren Schichten Feinstform-Absorberpigmente enthalten.

15. Abschirmung nach einem der Ansprüche 1 bis 14, **dadurch gekennzeichnet, daß** der Träger (10) in der Art von Radardomen oder Moskitonetzen selbsttragend ausgebildet ist und/oder mit Abstützungen einen z.B. zellenförmigen Abschirmbereich bildet.

16. Abschirmung nach einem der Ansprüche 1 bis 14, **dadurch gekennzeichnet, daß** der einen Membranstoff oder eine Vliesbahn aufweisende Träger (10) als Vorhang mit decken- und bodenseitigen Abschlüssen ausgebildet ist.

## Claims

1. Electromagnetic shielding for buildings, rooms, cabinets and the like, including a carrier (10) flat-attachable to the surface of room-enclosing walls (W, B, D), which carrier comprises a gluable fleece (12) as well as a continuous conductive layer (16) having an insulating strip coating (18) suited for attaching and again removing other layers, **wherein** the carrier (10) is designed as a rollable flat material in the form of gluable wallpaper webs and wherein at least one edge (20) has a preferably lengthwise perforated edge area (22) which is uncoated, i.e. free from a strip coating (18), so that the conductive layer (16) is bare.

2. Shielding according to claim 1, **wherein** the fleece (12) of the carrier (10) is provided with a flat primary coating (14) acting as insulation, to which the conductive layer (16) has been applied.

3. Shielding according to claim 1 or claim 2, **wherein** the conductive layer (16) consists of alloy powder, graphite or soot.

4. Shielding according to claims 2 and 3, **wherein** the conductive layer (16) consisting of an aqueous dispersion containing adhesives has been applied to the primary coating (14), e.g. by screen printing.

5. Shielding according to any one of claims 1 to 4, wherein neighbouring edge areas (22) of two webs (10) are adapted to be covered by a covering strip (30) of like layer structure so that the conductive layers (16, 36) are conductively connected with each other, in particular by direct material contact.

6. Shielding according to claim 5, **wherein** abutting web edges are adapted to be overlapped or are covered flushly by the covering strip (30).

7. Shielding according to claim 6, **wherein** the width of an edge strip (22) detachable along the perforation (24) is dimensioned so that as a covering strip (30), it laps over two abutting open web edges without gap.

8. Shielding according to any one of claims 1 to 7, **wherein** zones of the conductive layer (16) which are bare e.g. at edge strips (22) are suited to be or are conductively connected with flat flexible contact elements, especially in the area of apertures of the room to be shielded and/or at earthed sockets (40), e.g. of the power supply system or the telephone network.

9. Shielding according to claim 8, **wherein** the contact elements are designed as flat contact springs and/or as conductive sealing strips.

10. Shielding according to any one of claims 1 to 9, **wherein** the strip coating (18) has a smooth outer surface suited for pasting on, in particular, top wall papers (T) or posters.

11. Shielding according to any one of claims 1 to 10, **wherein** the carrier (10) may be reworked by hand, e.g. in damaged edge areas.

12. Shielding according to any one of claims 5 to 11, **wherein** between surface layers (16, 36), further layers comprising or consisting of metal alloy powder (17) are arranged.

13. Shielding according to claim 12, **wherein** the further layers contain chemically neutralized, corrosion-free metal alloy powder.

14. Shielding according to claim 12 or claim 13, **wherein** the further layers contain superfine absorbent pigments.

15. Shielding according to any one of claims 1 to 14, **wherein** the carrier (10) is self-supporting in the way of radar domes or mosquito nets, and/or - together with support elements - forms a shielding zone, e.g. of cell-shape.

16. Shielding according to any one of claims 1 to 14, **wherein** the carrier (10) is provided with a membrane cloth or a fleece web and is designed as a curtain having seams at the ceiling and the floor sides.

## Revendications

1. Blindage électromagnétique pour bâtiments, salles, armoires ou autres, avec un support (10) pouvant être apposé en surface sur des parois de limitation de l'espace (W, B, D) et disposant d'un voile collable (12) et d'une couche intégralement conductrice (16), laquelle porte un revêtement de pellicule isolante (18) sur lequel d'autres couches peuvent être apposées et ensuite retirées, **caractérisé en ce que** le support (10) est conçu en tant que matériel plat enroulable sous forme de pans de tapisserie collables, et **en ce que** le long d'au moins une arête (20) de pan, une zone marginale (22) de préférence perforée longitudinalement n'est pas revêtue, c'est-à-dire sans revêtement de pellicule (18), afin que la couche conductrice (16) soit découverte.

2. Blindage selon la revendication 1, **caractérisé en ce que** le voile (12) du support (10) est pourvu d'une couche de base plane (14) servant d'isolation, sur laquelle est apposée la couche conductrice (16).

3. Blindage selon la revendication 1 ou 2, **caractérisé en ce que** la couche conductrice (16) est en poudre d'alliage, graphite ou suie.

4. Blindage selon la revendication 2 ou 3, **caractérisé en ce que** la couche conductrice (16) en dispersion aqueuse contenant des agents adhésifs est appliquée sur la couche de base (14), par exemple par sérigraphie.

5. Blindage selon l'une des revendications 1 à 4, **caractérisé en ce que** des zones marginales voisines (22) de deux pans (10) peuvent être recouvertes de telle manière par une bande de recouvrement (30) d'une même structure des couches, que les couches conductrices (16, 36) sont raccordées pour la conduction, notamment par contact solidaire des matières.

6. Blindage selon la revendication 5, **caractérisé en ce que** des bords juxtaposés de pans peuvent être chevauchés ou recouverts à fleur par la bande de recouvrement (30).

7. Blindage selon la revendication 6, **caractérisé en ce que** la largeur d'une bande marginale (22) pouvant être détachée le long de la perforation (24) est dimensionnée de manière à pouvoir recouvrir complètement, en tant que bande de recouvrement (30), deux bords découverts de pans juxtaposés.

8. Blindage selon l'une des revendications 1 à 7, **caractérisé en ce que** des zones découvertes de la couche conductrice (16), par exemple sur les bandes marginales (22), sont ou peuvent être raccordées pour la conduction, au moyen d'éléments de contact plans et flexibles, notamment dans la zone des ouvertures de la pièce à blinder et/ou près de prises (40) mises à la terre, par exemple du secteur électrique ou du réseau téléphonique.

9. Blindage selon la revendication 8, **caractérisé en ce que** les éléments de contact sont conçus en tant que ressorts de contact plans et/ou en tant que bandes d'étanchement conductrices.

10. Blindage selon l'une des revendications 1 à 9, **caractérisé en ce que** le revêtement de pellicule (18) dispose d'une surface extérieure lisse, sur laquelle peuvent notamment être collés un papier peint de recouvrement (T) ou des affiches.

11. Blindage selon l'une des revendications 1 à 10, **caractérisé en ce que** le support (10) peut être soumis à des reprises manuelles, par exemple dans des zones marginales endommagées.

12. Blindage selon l'une des revendications 5 à 11, **caractérisé en ce que** d'autres couches en ou avec de la poudre d'alliage métallique (17) sont disposées entre des couches de recouvrement (16, 36).

13. Blindage selon la revendication 12, **caractérisé en ce que** les autres couches contiennent de la poudre d'alliage métallique neutralisée chimiquement et non corrosive.

14. Blindage selon la revendication 12 ou 13, **caractérisé en ce que** les autres couches contiennent des pigments absorbeurs de forme ultra-fine.

15. Blindage selon l'une des revendications 1 à 14, **caractérisé en ce que** le support (10) est conçu autoporteur à la manière des dômes de radar ou des moustiquaires, et/ou qu'il forme à l'aide de supports une zone blindée en forme de cellule par exemple.

16. Blindage selon l'une des revendications 1 à 14, **caractérisé en ce que** le support (10) disposant d'un tissu membraneux ou d'un pan de voile est conçu en tant que rideau, avec des lisières sur les côtés du sol et du plafond.
